(19) Europäisches Patentamt
European Patent Office
Office européen des brevets

(11) EP 3 170 844 A1

(12) **EUROPEAN PATENT APPLICATION**
published in accordance with Art. 153(4) EPC

(43) Date of publication:
**24.05.2017 Bulletin 2017/21**

(21) Application number: **15821542.6**

(22) Date of filing: **06.07.2015**

(51) Int Cl.:
***C08F 2/48*** *(2006.01)* ***C07D 295/10*** *(2006.01)*

(86) International application number:
**PCT/JP2015/069399**

(87) International publication number:
**WO 2016/009871 (21.01.2016 Gazette 2016/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **16.07.2014 JP 2014146032**

(71) Applicant: **Adeka Corporation**
**Tokyo 116-8554 (JP)**

(72) Inventors:
- **KANEHARA, Yukiko**
**Tokyo 116-8554 (JP)**
- **TAMACHI, Tomoya**
**Tokyo 116-8554 (JP)**
- **ARIYOSHI, Tomoyuki**
**Tokyo 116-8554 (JP)**

(74) Representative: **Forstmeyer, Dietmar et al**
**BOETERS & LIECK**
**Oberanger 32**
**80331 München (DE)**

# (54) PHOTOSENSITIVE COMPOSITION

(57) Provided is a photosensitive composition employing, as a photopolymerization initiator, a compound useful as a highly-sensitive photopolymerization initiator that has excellent stability and low sublimability, and that efficiently absorbs and is activated by near-ultraviolet rays at, for example, 365 nm. A photosensitive composition includes a photopolymerization initiator including General Formula (I) below (A), and a polymerizable compound having an ethylenically unsaturated bond (B). Preferred, among compounds represented by General Formula (I), are compounds in which $R^{10}$ is a hydrogen atom, or a $C_{1-12}$ alkyl group that is not substituted or that is substituted by a hydroxyl group, a carboxyl group, a halogen atom, a cyano group, or a nitro group.

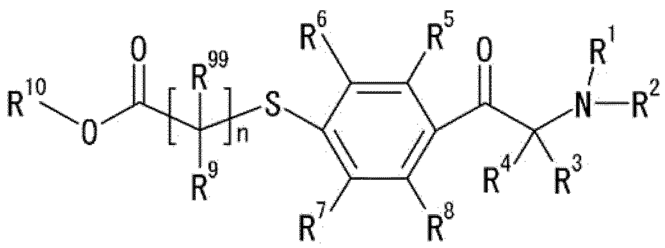

(I)



Printed by Jouve, 75001 PARIS (FR)

**Description**

Technical Field

The present invention relates to a photosensitive composition including a photopolymerization initiator and a polymerizable compound having an ethylenically unsaturated bond.

Background Art

Photosensitive compositions are prepared by adding a photopolymerization initiator to a polymerizable compound having at least one ethylenically unsaturated bond. Because such photosensitive compositions can be polymerized and cured by irradiation with energy rays (light), they are used, for example, in photosensitive printing plates and various types of photoresists.

Patent Literatures 1 and 2 propose the use of $\alpha$-aminoalkylphenone derivatives as photopolymerization initiators used for such photosensitive compositions.

Unfortunately, the $\alpha$-aminoalkylphenone derivatives disclosed in Patent Literatures 1 and 2 do not have sufficiently low sublimability, and sublimed products thereof cause contamination of photomasks and heating furnaces.

Citation List

Patent Literature

Patent Literature 1: US 4,321,118A
Patent Literature 2: JP 2007-086565A

Summary of Invention

One of the problems to be solved by the invention is that there has yet to be provided a photopolymerization initiator having satisfactory low sublimability.

Accordingly, the present invention aims at providing a photosensitive composition employing, as a photopolymerization initiator, a compound useful as a highly-sensitive photopolymerization initiator that has excellent stability and low sublimability, and that efficiently absorbs and is activated by near-ultraviolet rays at, for example, 365 nm.

Based on the above finding, the invention provides a photosensitive composition including a photopolymerization initiator including a compound represented by General Formula (I) below (A), and a polymerizable compound having an ethylenically unsaturated bond (B).

[Chem. 1]

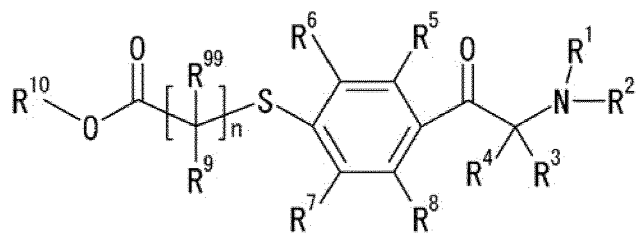

(I)

(wherein, $R^1$ and $R^2$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^1$ and $R^2$ may be connected together to form a 3- to 6-membered heterocycle;

$R^3$ and $R^4$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen

atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^3$ and $R^4$ may be connected together to form a 3- to 6-membered ring;

$R^5$, $R^6$, $R^7$, and $R^8$ each independently represent a hydrogen atom, a halogen atom, a cyano group, a nitro group, a hydroxyl group, or a $C_{1-8}$ alkyl group that is not substituted or that is substituted by a halogen atom;

$R^9$ and $R^{99}$ each independently represent a hydrogen atom, a hydroxyl group, a carboxyl group, a halogen atom, or a $C_{1-4}$ alkyl group, and the $R^9$s, and the $R^{99}$s, are the same or different from one another when n is 2 or greater;

$R^{10}$ represents a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; or a $C_{7-30}$ arylalkyl group that is not substituted or that is substituted by a $C_{1-4}$ alkyl group, a hydroxyl group, a carboxyl group, a halogen atom, a cyano group, or a nitro group;

a methylene chain in the alkyl group and the arylalkyl group may be replaced by -O- or -S-; and

n represents a number from 1 to 12.)

Also, the invention provides a cured product obtained by irradiating the above-mentioned photosensitive composition with energy rays.

Further, the invention provides a compound represented by General Formula (I') below.

[Chem. 2]

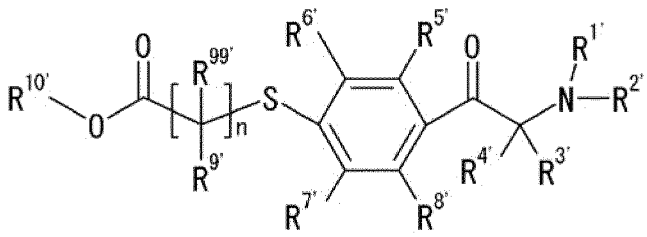

(I')

(wherein, $R^{1'}$ and $R^{2'}$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^{1'}$ and $R^{2'}$ may be connected together to form a 3- to 6-membered heterocycle;

$R^{3'}$ and $R^{4'}$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^{3'}$ and $R^{4'}$ may be connected together to form a 3- to 6-membered ring;

$R^{5'}$, $R^{6'}$, $R^{7'}$, and $R^{8'}$ each independently represent a hydrogen atom, a halogen atom, a cyano group, a nitro group, a hydroxyl group, or a $C_{1-8}$ alkyl group that is not substituted or that is substituted by a halogen atom;

$R^{9'}$ represents a hydroxyl group, a carboxyl group, a halogen atom, or a $C_{1-4}$ alkyl group, and the $R^{9'}$s are the same or different from one another when n is 2 or greater;

$R^{99'}$ represents a hydrogen atom, a hydroxyl group, a carboxyl group, a halogen atom, or a $C_{1-4}$ alkyl group, and the $R^{99'}$s are the same or different from one another when n is 2 or greater;

$R^{10'}$ represents a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; or a $C_{7-30}$ arylalkyl group that is not substituted or that is substituted by a $C_{1-4}$ alkyl group, a hydroxyl group, a carboxyl group, a halogen atom, a cyano group, or a nitro group;

a methylene chain in the alkyl group and the arylalkyl group may be replaced by -O- or -S-; and

n represents a number from 1 to 12.)

Description of Embodiments

Preferred embodiments of a compound according to the present invention and a photopolymerization initiator containing the compound are described in detail below.

Examples of non-substituted $C_{1-12}$ alkyl groups represented by $R^1$, $R^2$, $R^3$, $R^4$, and $R^{10}$ in the aforementioned General Formula (I) include methyl, ethyl, propyl, isopropyl, butyl, isobutyl, s-butyl, t-butyl, amyl, isoamyl, t-amyl, hexyl, heptyl, octyl, isooctyl, 2-ethylhexyl, t-octyl, nonyl, isononyl, decyl, isodecyl, undecyl, dodecyl, cyclopentyl, cyclopentylmethyl, cyclopentylethyl, cyclohexyl, cyclohexyl methyl, and cyclohexyl ethyl.

Examples of $C_{1-12}$ alkyl groups substituted by a hydroxyl group, a carboxyl group, a halogen atom, a cyano group, or a nitro group, as represented by $R^1$, $R^2$, $R^3$, $R^4$, and $R^{10}$ in the aforementioned General Formula (I), include groups in which a portion or all of the hydrogen atoms in the aforementioned $C_{1-12}$ alkyl group is/are substituted by a hydroxyl group, a carboxyl group, a halogen atom, a cyano group, or a nitro group.

Examples of non-substituted $C_{1-8}$ alkyl groups represented by $R^5$, $R^6$, $R^7$, and $R^8$ in the aforementioned General Formula (I) include $C_{1-8}$ alkyl groups among the alkyl groups given as examples for the aforementioned non-substituted $C_{1-12}$ alkyl groups.

Examples of $C_{1-8}$ alkyl groups substituted by a halogen atom, as represented by $R^5$, $R^6$, $R^7$, and $R^8$ in the aforementioned General Formula (I), include groups in which a portion or all of the hydrogen atoms in the aforementioned $C_{1-8}$ alkyl group is/are substituted by a halogen atom.

Examples of $C_{1-4}$ alkyl groups represented by $R^9$ and $R^{99}$ in the aforementioned General Formula (I) include $C_{1-4}$ alkyl groups among the alkyl groups given as examples for the aforementioned non-substituted $C_{1-12}$ alkyl groups.

Examples of non-substituted $C_{7-30}$ arylalkyl groups represented by $R^1$, $R^2$, $R^3$, $R^4$, and $R^{10}$ in the aforementioned General Formula (I) include benzyl, α-methylbenzyl, α,α-dimethylbenzyl, and phenylethyl.

Examples of $C_{7-30}$ arylalkyl groups substituted by a $C_{1-4}$ alkyl group, a hydroxyl group, a carboxyl group, a halogen atom, a cyano group, or a nitro group, as represented by $R^1$, $R^2$, $R^3$, $R^4$, and $R^{10}$, include $C_{7-30}$ arylalkyl groups substituted by a $C_{1-4}$ alkyl group, a hydroxyl group, a carboxyl group, a halogen atom, a cyano group, or a nitro group, and include, for example, arylalkyl groups in which a portion or all of the hydrogen atoms in the aforementioned $C_{7-30}$ arylalkyl group is/are substituted by a hydroxyl group, a carboxyl group, a halogen atom, a cyano group, or a nitro group.

Examples of $C_{2-12}$ alkenyl groups represented by $R^1$, $R^2$, $R^3$, and $R^4$ in the aforementioned General Formula (I) include a vinyl group, a 1-propenyl group, a 2-propenyl group, an isopropenyl group, a 1-butenyl group, a 2-butenyl group, a 3-butenyl group, a 1-octenyl group, and a 1-decenyl group.

Examples of 3- to 6-membered heterocycles that may be formed by connecting together $R^1$ and $R^2$ in the aforementioned General Formula (I) preferably include a piperidine ring, a piperazine ring, a morpholine ring, and a lactam ring.

Examples of 3- to 6-membered rings that may be formed by connecting together $R^3$ and $R^4$ in the aforementioned General Formula (I) preferably include a cyclopentane ring, a cyclohexane ring, a cyclopentene ring, a lactone ring, and a lactam ring.

Examples of halogen atoms that may substitute $R^1$, $R^2$, $R^3$, $R^4$, $R^5$, $R^6$, $R^7$, $R^8$, $R^9$, $R^{10}$, and $R^{99}$ in the aforementioned General Formula (I) include fluorine, chlorine, bromine, and iodine.

Examples of alkyl groups that may substitute $R^1$, $R^2$, $R^3$, $R^4$, $R^9$, $R^{10}$, and $R^{99}$ in the aforementioned General Formula (I) include $C_{1-4}$ alkyl groups among the alkyl groups given as examples for the aforementioned non-substituted $C_{1-12}$ alkyl groups.

Among compounds represented by the aforementioned General Formula (I), compounds in which $R^{10}$ is a hydrogen atom or a $C_{1-12}$ alkyl group that is not substituted or that is substituted by a hydroxyl group, a carboxyl group, a halogen atom, a cyano group, or a nitro group are preferable because such compounds have low sublimability and are easy to produce.

Accordingly, preferred concrete examples of compounds represented by General Formula (I) include the following Compounds Nos. 1 to 30, although the present invention is not limited thereto.

[Chem. 3]

Compound No. 1

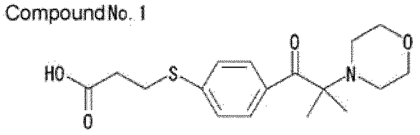

Compound No. 2

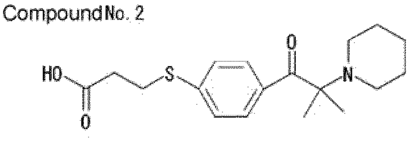

Compound No. 3

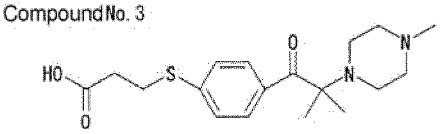

Compound No. 4

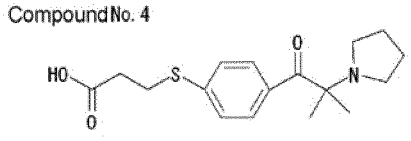

Compound No. 5

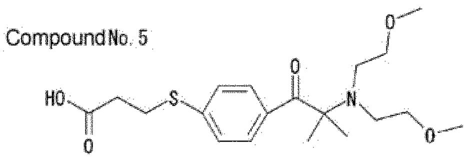

Compound No. 6

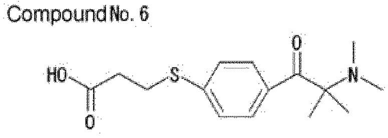

Compound No. 7

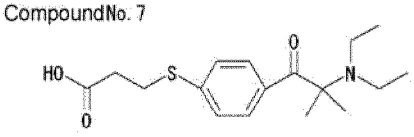

Compound No. 8

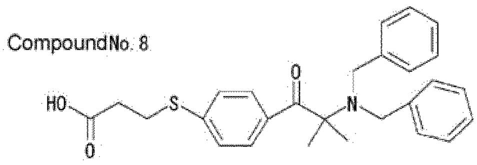

Compound No. 9

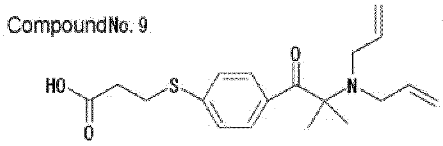

Compound No. 10

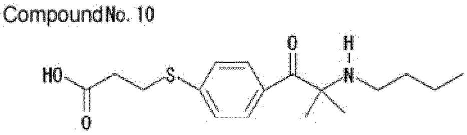

Compound No. 11

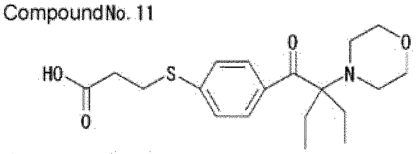

Compound No. 12

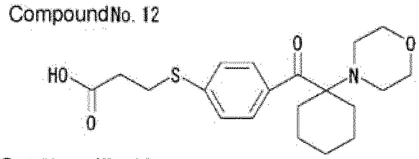

Compound No. 13

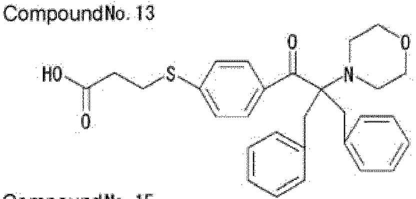

Compound No. 14

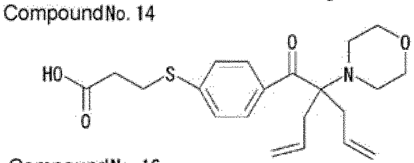

Compound No. 15

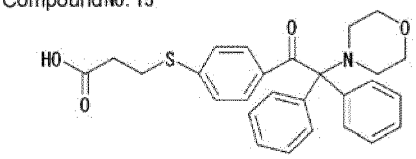

Compound No. 16

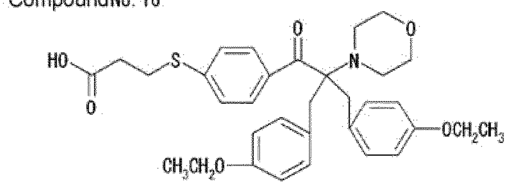

[Chem. 4]

Compound No. 17

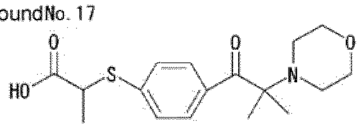

Compound No. 18

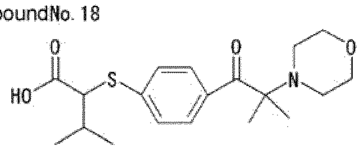

Compound No. 19

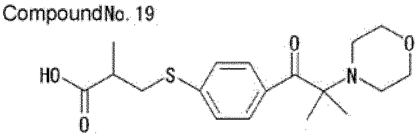

Compound No. 20

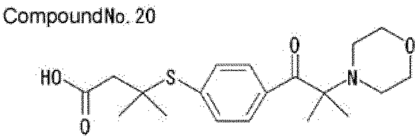

Compound No. 21

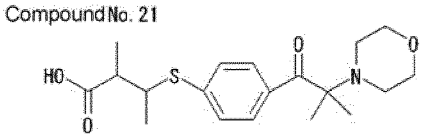

Compound No. 22

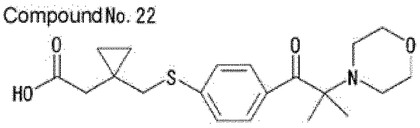

Compound No. 23

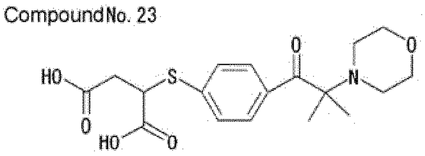

Compound No. 24

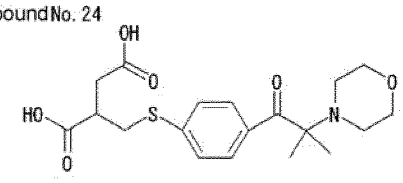

Compound No. 25

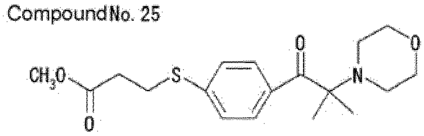

Compound No. 26

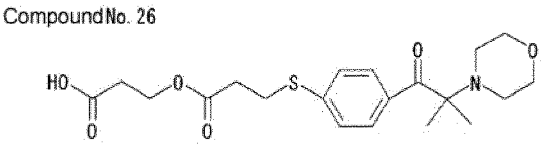

Compound No. 27

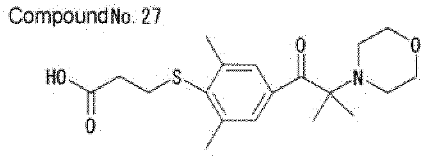

Compound No. 28

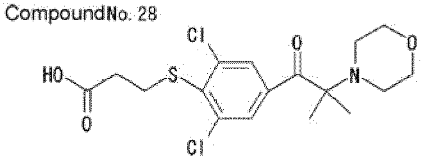

Compound No. 29

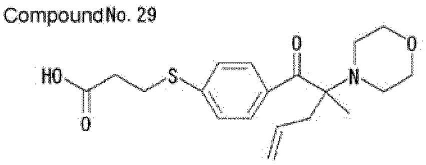

Compound No. 30

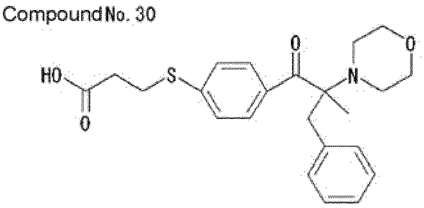

The photopolymerization initiator employed in the present invention contains at least one type of compound represented by the aforementioned General Formula (I), and is particularly useful as a photopolymerization initiator for a polymerizable compound having an ethylenically unsaturated bond. The content of the compound represented by the aforementioned General Formula (I) is preferably from 30 to 100% by mass, more preferably from 50 to 100% by mass, with respect to the photopolymerization initiator employed in the present invention.

The photosensitive composition of the present invention contains, as essential components, the polymerization initiator represented by the aforementioned General Formula (I) and a polymerizable compound having an ethylenically unsaturated bond, and also contains, in combination, optional components, such as an alkali-developable compound optionally having an ethylenically unsaturated group, an inorganic compound, a colorant, and a solvent.

Any ethylenically unsaturated polymerizable compound that has heretofore been used in photosensitive compositions can be used without particular limitation as the polymerizable compound having an ethylenically unsaturated bond. Examples include: unsaturated aliphatic hydrocarbons, such as ethylene, propylene, butylene, isobutylene, vinyl chloride, vinylidene chloride, vinylidene fluoride, and tetrafluoroethylene; (meth)acrylic acid, α-chloroacrylic acid, itaconic

acid, maleic acid, citraconic acid, fumaric acid, hymic acid, crotonic acid, isocrotonic acid, vinylacetic acid, allylacetic acid, cinnamic acid, sorbic acid, mesaconic acid, mono[2-(meth)acryloyloxyethyl] succinate, mono[2-(meth)acryloyloxyethyl] phthalate, a mono(meth)acrylate of a polymer having a carboxy group and a hydroxyl group at both ends, such as ω-carboxypolycaprolactone mono(meth)acrylate; hydroxyethyl (meth)acrylate-maleate, hydroxypropyl (meth)acrylate-maleate, dicyclopentadiene-maleate, unsaturated polybasic acids such as a polyfunctional (meth)acrylate having one carboxyl group and two or more (meth)acryloyl groups; 2-hydroxyethyl (meth)acrylate, 2-hydroxypropyl (meth)acrylate, glycidyl (meth)acrylate, Compounds Nos. A1 to A4 shown below, methyl (meth)acrylate, butyl (meth)acrylate, isobutyl (meth)acrylate, t-butyl (meth)acrylate, cyclohexyl (meth)acrylate, n-octyl (meth)acrylate, isooctyl (meth)acrylate, isononyl (meth)acrylate, stearyl (meth)acrylate, lauryl (meth)acrylate, methoxyethyl (meth)acrylate, dimethylaminomethyl (meth)acrylate, dimethylaminoethyl (meth)acrylate, aminopropyl (meth)acrylate, dimethylaminopropyl (meth)acrylate, ethoxyethyl (meth)acrylate, poly(ethoxy)ethyl (meth)acrylate, butoxyethoxyethyl (meth)acrylate, ethylhexyl (meth)acrylate, phenoxyethyl (meth)acrylate, tetrahydrofuryl (meth)acrylate, vinyl (meth)acrylate, allyl (meth)acrylate, benzyl (meth)acrylate; esters between an unsaturated monobasic acid and a polyhydric alcohol or polyhydric phenol, such as ethylene glycol di(meth)acrylate, diethylene glycol di(meth)acrylate, triethylene glycol di(meth)acrylate, polyethylene glycol di(meth)acrylate, propylene glycol di(meth)acrylate, 1,4-butanediol di(meth)acrylate, 1,6-hexanediol di(meth)acrylate, trimethylolethane tri(meth)acrylate, trimethylolpropane tri(meth)acrylate, dipentaerythritol hexa(meth)acrylate, dipentaerythritol penta(meth)acrylate, pentaerythritol tetra(meth)acrylate, pentaerythritol tri(meth)acrylate, tricyclodecanedimethylol di(meth)acrylate, tri[(meth)acryloylethyl] isocyanurate, and polyester (meth)acrylate oligomers; metal salts of unsaturated polybasic acids, such as zinc (meth)acrylate and magnesium (meth)acrylate; unsaturated polybasic acid anhydrides, such as maleic anhydride, itaconic anhydride, citraconic anhydride, methyltetrahydrophthalic anhydride, tetrahydrophthalic anhydride, trialkyltetrahydrophthalic anhydrides, 5-(2,5-dioxotetrahydrofuryl)-3-methyl-3-cyclohexene-1,2-dicarboxylic acid anhydride, trialkyltetrahydrophthalic anhydride-maleic anhydride adducts, dodecenylsuccinic anhydride, and methylhymic anhydride; amides formed between an unsaturated monobasic acid and a polyfunctional amine, such as (meth)acrylamide, methylenebis(meth)acrylamide, diethylenetriaminetris(meth)acrylamide, xylylenebis(meth)acrylamide, α-chloroacrylamide, and N-2-hydroxyethyl (meth)acrylamide; unsaturated aldehydes, such as acrolein; unsaturated nitriles, such as (meth)acrylonitrile, α-chloroacrylonitrile, vinylidene cyanide, and allyl cyanide; unsaturated aromatic compounds, such as styrene, 4-methylstyrene, 4-ethylstyrene, 4-methoxystyrene, 4-hydroxystyrene, 4-chlorostyrene, divinylbenzene, vinyltoluene, vinylbenzoic acid, vinylphenol, vinylsulfonic acid, 4-vinylbenzenesulfonic acid, vinylbenzyl methyl ether, and vinylbenzyl glycidyl ether; unsaturated ketones, such as methyl vinyl ketone; unsaturated amine compounds, such as vinylamine, allylamine, N-vinylpyrrolidone, and vinylpiperidine; vinyl alcohols, such as allyl alcohol and crotyl alcohol; vinyl ethers, such as vinyl methyl ether, vinyl ethyl ether, n-butyl vinyl ether, isobutyl vinyl ether, and allyl glycidyl ether; unsaturated imides, such as maleimide, N-phenylmaleimide, and N-cyclohexylmaleimide; indenes, such as indene and 1-methylindene; aliphatic conjugated dienes, such as 1,3-butadiene, isoprene, and chloroprene; macromonomers having a mono(meth)acryloyl group at the end of the polymeric molecular chain thereof, such as polystyrene, polymethyl (meth)acrylate, poly-n-butyl (meth)acrylate, and polysiloxanes; vinyl chloride, vinylidene chloride, divinyl succinate, diallyl phthalate, triallyl phosphate, triallyl isocyanurate, vinyl thioether, vinylimidazole, vinyloxazoline, vinylcarbazole, vinylpyrrolidone, vinylpyridine, vinylurethane compounds formed between a hydroxyl-containing vinyl monomer and a polyisocyanate compound, and vinylepoxy compounds formed between a hydroxyl-containing vinyl monomer and a polyepoxy compound.

Among the aforementioned compounds, the photopolymerization initiator including a compound represented by the aforementioned General Formula (I) is suitable for mono(meth)acrylates of polymers having a carboxy group and a hydroxyl group at respective ends, polyfunctional (meth)acrylates having one carboxy group and two or more (meth)acryloyl groups, and esters between an unsaturated monobasic acid and a polyhydric alcohol or polyhydric phenol.

The polymerizable compounds can be used either alone or as a mixture containing two or more types of compounds. Where two or more types of compounds are to be used in combination, they may be copolymerized in advance to form a copolymer.

[Chem. 5]

Compound No. A1

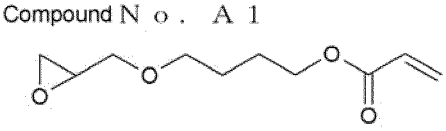

[Chem. 6]

Compound No. A2

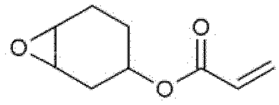

[Chem. 7]

Compound No. A3

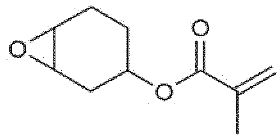

[Chem. 8]

Compound No. A4

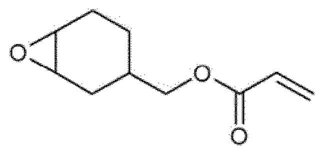

The aforementioned alkali-developable compounds optionally having an ethylenically unsaturated group are not particularly limited as long as they are soluble in an alkaline aqueous solution, and examples include resins etc. disclosed, for example, in JP 2004-264414A.

Examples of resins that can be used as alkali-developable compounds optionally having an ethylenically unsaturated group include: acrylic ester copolymers; phenol and/or cresol novolac epoxy resins; polyphenylmethane epoxy resins having polyfunctional epoxy groups; epoxy acrylate resins; and resins obtained by reacting an epoxy group in, for example, an epoxy compound represented by the following General Formula (II), with an unsaturated monobasic acid followed by reaction with a polybasic acid anhydride.

Preferred among the above are resins obtained by reacting an epoxy group in, for example, an epoxy compound represented by the following General Formula (II), with an unsaturated monobasic acid followed by reaction with a polybasic acid anhydride.

It is also preferable that the alkali-developable compound optionally having an ethylenically unsaturated bond contains from 0.2 to 1.0 equivalents of unsaturated groups.

[Chem. 9]

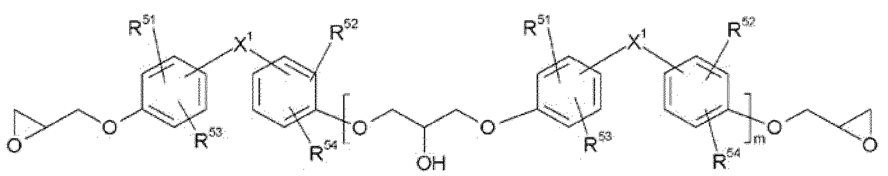

(II)

(wherein, $X^1$ represents a direct bond, a methylene group, a $C_{1-4}$ alkylidene group, a $C_{3-20}$ alicyclic hydrocarbon group, O, S, $SO_2$, SS, SO, CO, OCO, or a group represented by the following Chem. 10, Chem. 11, or Chem. 12; the alkylidene group may optionally be substituted by a halogen atom; $R^{51}$, $R^{52}$, $R^{53}$, and $R^{54}$ each independently represent a hydrogen atom, a $C_{1-5}$ alkyl group, a $C_{1-8}$ alkoxy group, a $C_{2-5}$ alkenyl group, or a halogen atom; the alkyl group, the alkoxy group, and the alkenyl group may optionally be substituted by a halogen atom; m is an integer of 0 to 10; and when m is not 0, the optical isomer may be any isomer.)

[Chem. 10]

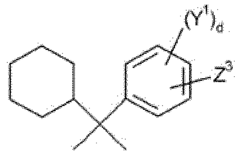

(wherein, $Z^3$ represents a hydrogen atom, a phenyl group optionally substituted by a $C_{1-10}$ alkyl group or a $C_{1-10}$ alkoxy group, or a $C_{3-10}$ cycloalkyl group optionally substituted by a $C_{1-10}$ alkyl group or a $C_{1-10}$ alkoxy group; $Y^1$ represents a $C_{1-10}$ alkyl group, a $C_{1-10}$ alkoxy group, a $C_{2-10}$ alkenyl group, or a halogen atom; the alkyl group, the alkoxy group, and the alkenyl group may optionally be substituted by a halogen atom; and d is an integer of 0 to 5.)

[Chem. 11]

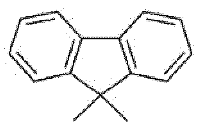

[Chem. 12]

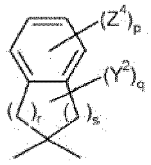

(wherein, $Y^2$ and $Z^4$ each independently represent a $C_{1-10}$ alkyl group optionally substituted by a halogen atom, a $C_{6-20}$ aryl group optionally substituted by a halogen atom, a $C_{6-20}$ aryloxy group optionally substituted by a halogen atom, a $C_{6-20}$ arylthio group optionally substituted by a halogen atom, a $C_{6-20}$ arylalkenyl group optionally substituted by a halogen atom, a $C_{7-20}$ arylalkyl group optionally substituted by a halogen atom, a $C_{2-20}$ heterocyclic group optionally substituted by a halogen atom, or a halogen atom; the alkylene portion in the alkyl group and the arylalkyl group may be interrupted by an unsaturated bond, -O-, or -S-; $Z^4$ may form a ring with an adjacent $Z^4$; p is an integer of 0 to 4; q is an integer of 0 to 8; r is an integer of 0 to 4; s is an integer of 0 to 4; and the sum of r and s is an integer of 2 to 4.)

Examples of the unsaturated monobasic acids for reaction with the epoxy compound include acrylic acid, methacrylic acid, crotonic acid, cinnamic acid, sorbic acid, hydroxyethyl methacrylate-maleate, hydroxyethyl acrylate-maleate, hydroxypropyl methacrylate-maleate, hydroxypropyl acrylate-maleate, and dicyclopentadiene-maleate.

Examples of the polybasic acid anhydrides for reaction after the reaction of the unsaturated monobasic acid include biphenyltetracarboxylic acid dianhydride, tetrahydrophthalic anhydride, succinic anhydride, biphthalic anhydride, maleic anhydride, trimellitic anhydride, pyromellitic anhydride, 2,2',3,3'-benzophenonetetracarboxylic acid anhydride, ethylene glycol bisanhydrotrimellitate, glycerol trisanhydrotrimellitate, hexahydrophthalic anhydride, methyltetrahydrophthalic anhydride, nadic anhydride, methylnadic anhydride, trialkyltetrahydrophthalic anhydrides, hexahydrophthalic anhydride, 5-(2,5-dioxotetrahydrofuryl)-3-methyl-3-cyclohexene-1,2-dicarboxylic acid anhydride, trialkyltetrahydrophthalic anhydride-maleic anhydride adducts, dodecenylsuccinic anhydride, and methylhymic anhydride.

Preferably, the molar ratio on reaction among the epoxy compound, the unsaturated monobasic acid, and the polybasic acid anhydride is as follows.

An epoxy adduct formed by reacting the epoxy compound and the unsaturated monobasic acid preferably has a structure wherein from 0.1 to 1.0 carboxyl groups of the unsaturated monobasic acid is added per one epoxy group of the epoxy compound. The polybasic acid anhydride is used in such a molar ratio as to preferably provide from 0.1 to 1.0 acid anhydride structure per one hydroxyl group of the above epoxy adduct.

The reactions among the epoxy compound, the unsaturated monobasic acid, and the polybasic acid anhydride can be carried out according to ordinary methods.

The alkali-developable photosensitive resin composition of the present invention, which is an embodiment of the photosensitive composition of the invention, contains, as essential components, the photopolymerization initiator of the invention, a polymerizable compound having an ethylenically unsaturated bond, and an alkali-developable compound optionally having an ethylenically unsaturated group, and may also contain, in combination, optional components such as an inorganic compound, a colorant, and a solvent. Note that an alkali-developable photosensitive resin composition

of the invention containing a colorant is also referred to particularly as an "alkali-developable, colored photosensitive resin composition" of the invention.

The above-described polymerizable compound having an ethylenically unsaturated bond and the alkali-developable compound optionally having an ethylenically unsaturated group may be the same compound or may be different compounds, and they may be used either alone or as a mixture containing two or more types of compounds.

In order to improve the developability of the alkali-developable (colored) photosensitive resin composition of the invention, the alkali-developable (colored) photosensitive resin composition of the invention may further contain a mono- or polyfunctional epoxy compound in combination with the above-described alkali-developable compound optionally having an ethylenically unsaturated bond. It is preferable that the acid value of the solids content of the above-described alkali-developable compound optionally having an ethylenically unsaturated bond is within a range of from 5 to 120 mg-KOH/g, and accordingly, the amount of the mono- or polyfunctional epoxy compound to be used is preferably determined so as to satisfy the above acid value range.

Examples of the monofunctional epoxy compounds include glycidyl methacrylate, methyl glycidyl ether, ethyl glycidyl ether, propyl glycidyl ether, isopropyl glycidyl ether, butyl glycidyl ether, isobutyl glycidyl ether, t-butyl glycidyl ether, pentyl glycidyl ether, hexyl glycidyl ether, heptyl glycidyl ether, octyl glycidyl ether, nonyl glycidyl ether, decyl glycidyl ether, undecyl glycidyl ether, dodecyl glycidyl ether, tridecyl glycidyl ether, tetradecyl glycidyl ether, pentadecyl glycidyl ether, hexadecyl glycidyl ether, 2-ethylhexyl glycidyl ether, allyl glycidyl ether, propargyl glycidyl ether, p-methoxyethyl glycidyl ether, phenyl glycidyl ether, p-methoxy glycidyl ether, p-butylphenyl glycidyl ether, cresyl glycidyl ether, 2-methylcresyl glycidyl ether, 4-nonylphenyl glycidyl ether, benzyl glycidyl ether, p-cumylphenyl glycidyl ether, trityl glycidyl ether, 2,3-epoxypropyl methacrylate, epoxidized soybean oil, epoxidized linseed oil, glycidyl butyrate, vinylcyclohexane monoxide, 1,2-epoxy-4-vinylcyclohexane, styrene oxide, pinene oxide, methylstyrene oxide, cyclohexene oxide, propylene oxide, and Compounds Nos. E1 and E2 below.

[Chem. 13]

Compound No. E1

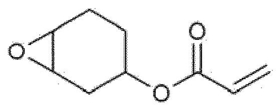

[Chem. 14]

Compound No. E2

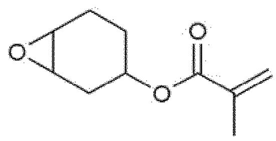

It is preferable to use at least one type of compound selected from the group consisting of bisphenol epoxy compounds and glycidyl ethers as the polyfunctional epoxy compound, because this allows to provide an alkali-developable (colored) photosensitive resin composition having further improved characteristics.

Examples of usable bisphenol epoxy compounds include the epoxy compounds represented by the aforementioned General Formula (II) and other bisphenol epoxy compounds, such as hydrogenated bisphenol epoxy compounds.

Examples of usable glycidyl ethers include ethylene glycol diglycidyl ether, propylene glycol diglycidyl ether, 1,4-butanediol diglycidyl ether, 1,6-hexanediol diglycidyl ether, 1,8-octanediol diglycidyl ether, 1,10-decanediol diglycidyl ether, 2,2-dimethyl-1,3-propanediol diglycidyl ether, diethylene glycol diglycidyl ether, triethylene glycol diglycidyl ether, tetraethylene glycol diglycidyl ether, hexaethylene glycol diglycidyl ether, 1,4-cyclohexanedimethanol diglycidyl ether, 1,1,1-tri(glycidyloxymethyl)propane, 1,1,1-tri(glycidyloxymethyl)ethane, 1,1,1-tri(glycidyloxymethyl)methane, and 1,1,1,1-tetra(glycidyloxymethyl)methane.

Other usable polyfunctional epoxy compounds include: novolac epoxy compounds, such as phenol novolac epoxy compounds, biphenyl novolac epoxy compounds, cresol novolac epoxy compounds, bisphenol A novolac epoxy compounds, and dicyclopentadiene novolac epoxy compounds; alicyclic epoxy compounds, such as 3,4-epoxy-6-methylcyclohexylmethyl-3,4-epoxy-6-methylcyclohexanecarboxylate, 3,4-epoxycyclohexylmethyl-3,4-epoxycyclohexanecarboxylate, and 1-epoxyethyl-3,4-epoxycyclohexane; glycidyl esters, such as diglycidyl phthalate, diglycidyl tetrahydrophthalate, and glycidyl dimerate; glycidylamines, such as tetraglycidyl diaminodiphenylmethane, triglycidyl p-aminophenol, and N,N-diglycidylaniline; heterocyclic epoxy compounds, such as 1,3-diglycidyl-5,5-dimethylhydantoin and triglycidyl isocyanurate; dioxide compounds, such as dicyclopentadiene dioxide; naphthalene epoxy compounds; triphenylmethane epoxy compounds; and dicyclopentadiene epoxy compounds.

In the photosensitive composition of the present invention, the content of the photopolymerization initiator represented by the aforementioned General Formula (I) is not particularly limited, but is preferably from 0.1 to 70 parts by mass, more preferably from 1 to 50 parts by mass, even more preferably from 5 to 30 parts by mass, with respect to 100 parts by mass of the polymerizable compound having an ethylenically unsaturated bond.

Particularly in cases where the photosensitive composition of the invention is an alkali-developable (colored) photosensitive resin composition, the content of the alkali-developable compound optionally having an ethylenically unsaturated bond is preferably from 1 to 20% by mass, more preferably from 3 to 12% by mass, with respect to the alkali-developable (colored) photosensitive resin composition of the invention.

The photosensitive composition of the invention may optionally contain a solvent. Usually, solvents capable of dissolving or dispersing the above-described components (such as the photopolymerization initiator of the invention, the polymerizable compound having an ethylenically unsaturated bond, etc.) are used where necessary. Examples of such solvents include: ketones, such as methyl ethyl ketone, methyl amyl ketone, diethyl ketone, acetone, methyl isopropyl ketone, methyl isobutyl ketone, cyclohexanone, and 2-heptanone; ether-based solvents, such as ethyl ether, dioxane, tetrahydrofuran, 1,2-dimethoxyethane, 1,2-diethoxyethane, and dipropylene glycol dimethyl ether; ester-based solvents, such as methyl acetate, ethyl acetate, n-propyl acetate, isopropyl acetate, n-butyl acetate, cyclohexyl acetate, ethyl lactate, dimethyl succinate, and Texanol; cellosolve-based solvents, such as ethylene glycol monomethyl ether and ethylene glycol monoethyl ether; alcohol-based solvents, such as methanol, ethanol, iso- or n-propanol, iso- or n-butanol, and amyl alcohol; ether ester-based solvents, such as ethylene glycol monomethyl ether acetate, ethylene glycol monoethyl ether acetate, propylene glycol 1-monomethyl ether 2-acetate, dipropylene glycol monomethyl ether acetate, 3-methoxybutyl ether acetate, and ethoxyethyl ether propionate; BTX solvents, such as benzene, toluene, xylene, etc.; aliphatic hydrocarbon-based solvents, such as hexane, heptane, octane, and cyclohexane; terpene hydrocarbon oils, such as turpentine oil, D-limonene, and pinene; paraffinic solvents, such as mineral spirit, Swasol #310 (available from Cosmo Matsuyama Oil Co., Ltd.), and Solvesso #100 (available from Exxon Chemical); halogenated aliphatic hydrocarbon-based solvents, such as carbon tetrachloride, chloroform, trichloroethylene, methylene chloride, and 1,2-dichloroethane; halogenated aromatic hydrocarbon-based solvents, such as chlorobenzene; carbitol solvents; aniline; triethylamine; pyridine; acetic acid; acetonitrile; carbon disulfide; N,N-dimethylformamide; N,N-dimethylacetamide; N-methylpyrrolidone; dimethyl sulfoxide; and water. One type of solvent may be used alone, or two or more types may be used as a mixed solvent.

Among the above, solvents such as ketones and ether esters, and particularly propylene glycol 1-monomethyl ether 2-acetate, cyclohexanone, etc., are preferred because of their good compatibility with resists and photopolymerization initiators in the photosensitive composition.

The photosensitive composition of the invention (particularly the alkali-developable photosensitive resin composition) may further contain a colorant to be formulated into a colored (alkali-developable) photosensitive composition. Examples of colorants include pigments, dyes, and naturally-occurring colorants. The colorants may be used either alone or as a mixture containing two or more types of colorants.

Organic or inorganic pigments may be used, and examples of pigments include: nitroso compounds; nitro compounds; azo compounds; diazo compounds; xanthene compounds; quinoline compounds; anthraquinone compounds; coumarin compounds; phthalocyanine compounds; isoindolinone compounds; isoindoline compounds; quinacridone compounds; anthanthrone compounds; perynone compounds; perylene compounds; diketopyrrolopyrrole compounds; thioindigo compounds; dioxazine compounds; triphenylmethane compounds; quinophthalone compounds; naphthalenetetracarboxylic acids; metal complex compounds, such as azo dyes and cyanine dyes; lake pigments; carbon black species produced by the furnace, channel, or thermal process, and other carbon black species, such as acetylene black, Ketjen black, and lamp black; the above-mentioned carbon black adjusted or covered with an epoxy resin; the above-mentioned carbon black dispersed in advance with a resin in a solvent so as to adsorb 20 to 200 mg/g of the resin; the above-mentioned carbon black surface-treated with an acid or an alkali; the above-mentioned carbon black having an average particle size of 8 nm or above and a DBP oil absorption of 90 ml/100 g or less; the above-described carbon black whose total oxygen amount derived from the amount of CO and $CO_2$ in volatilized substances at 950°C is 9 mg or more per 100 $m^2$ of the carbon black's surface area; graphite, graphitized carbon black, activated carbon, carbon fiber, carbon nanotubes, carbon microcoils, carbon nanohorns, carbon aerogel, fullerene; aniline black, pigment black 7, titanium black; chromium oxide green, Milori blue, cobalt green, cobalt blue, manganese compounds, ferrocyanides, phosphate ultramarine blue, Prussian blue, ultramarine, cerulean blue, viridian, emerald green, lead sulfate, lead yellow, zinc yellow, Bengal red (red iron (III) oxide), cadmium red, synthetic iron black, amber, and extender pigments. The pigments may be used either alone or as a mixture of a plurality of pigments.

Commercially-available pigments may be used, and examples of such pigments include: pigment red 1, 2, 3, 9, 10, 14, 17, 22, 23, 31, 38, 41, 48, 49, 88, 90, 97, 112, 119, 122, 123, 144, 149, 166, 168, 169, 170, 171, 177, 179,

180, 184, 185, 192, 200, 202, 209, 215, 216, 217, 220, 223, 224, 226, 227, 228, 240, and 254; pigment orange 13, 31, 34, 36, 38, 43, 46, 48, 49, 51, 52, 55, 59, 60, 61, 62, 64, 65, and 71; pigment yellow 1, 3, 12, 13, 14, 16, 17, 20, 24, 55, 60, 73, 81, 83, 86, 93, 95, 97, 98, 100, 109, 110, 113, 114, 117, 120, 125, 126, 127, 129, 137, 138, 139, 147, 148, 150, 151, 152, 153, 154, 166, 168, 175, 180, and 185; pigment green 7, 10, and 36; pigment blue 15, 15:1, 15:2, 15:3, 15:4, 15:5, 15:6, 22, 24, 56, 60, 61, 62, and 64; and pigment violet 1, 19, 23, 27, 29, 30, 32, 37, 40, and 50.

Examples of dyes include azo dyes, anthraquinone dyes, indigoid dyes, triarylmethane dyes, xanthene dyes, alizarine dyes, acridine dyes, stilbene dyes, thiazole dyes, naphthol dyes, quinoline dyes, nitro dyes, indamine dyes, oxazine dyes, phthalocyanine dyes, and cyanine dyes. Several dyes may be used as a mixture.

In the photosensitive composition of the invention, the colorant content is preferably from 50 to 350 parts by mass, more preferably from 100 to 250 parts by mass, with respect to 100 parts by mass of the polymerizable compound having an ethylenically unsaturated bond.

The photosensitive composition of the invention may further contain an inorganic compound. Examples of inorganic compounds include: metal oxides, such as nickel oxide, iron oxide, iridium oxide, titanium oxide, zinc oxide, magnesium oxide, calcium oxide, potassium oxide, silica, and alumina; layered clay minerals, Milori blue, calcium carbonate, magnesium carbonate, cobalt compounds, manganese compounds, glass powder (especially glass frit), mica, talc, kaolin, ferrocyanides, various metal sulfates, sulfides, selenides, aluminum silicate, calcium silicate, aluminum hydroxide, platinum, gold, silver, and copper.

Preferred among the above are, for example, glass frit, titanium oxide, silica, layered clay minerals, and silver. In the photosensitive composition of the invention, the inorganic compound content is preferably from 0.1 to 1000 parts by mass, more preferably from 10 to 800 parts by mass, with respect to 100 parts by mass of the polymerizable compound having an ethylenically unsaturated bond. Note that one type of inorganic compound may be used alone, or two or more types may be used in combination.

The inorganic compounds are used as, for example, fillers, antireflection agents, electrically-conductive agents, stabilizers, flame retardants, mechanical strength improving agents, specific wavelength absorbing agents, or ink repellent agents.

The photosensitive composition of the invention may also contain a dispersing agent for dispersing the colorant and/or the inorganic compound. Any dispersing agent may be used without limitation as long as it can disperse and stabilize the colorant or the inorganic compound, and commercially-available dispersing agents, such as the BYK series available from BYK-Chemie, may be used. Particularly, polymeric dispersing agents composed of polyester, polyether, or polyurethane having a basic functional group, and dispersing agents having a nitrogen-atom-containing basic functional group and wherein the nitrogen-containing functional group is an amine and/or its quaternary salt and the amine value is from 1 to 100 mg-KOH/g may suitably be used.

The photosensitive composition of the present invention may contain other photopolymerization initiators to be used in combination with the photopolymerization initiator represented by the aforementioned General Formula (I). Various known compounds may be used as the other photopolymerization initiator(s) to be used in combination, and examples include: benzophenone, phenyl biphenyl ketone, 1-hydroxy-1-benzoylcyclohexane, benzoin, benzyl dimethyl ketal, 1-benzyl-1-dimethylamino-1-(4'-morpholinobenzoyl)propane, 2-morpholyl-2-(4'-methylmercapto)benzoylpropane, thioxanthone, 1-chloro-4-propoxythioxanthone, isopropylthioxanthone, diethylthioxanthone, ethylanthraquinone, 4-benzoyl-4'-methyldiphenyl sulfide, benzoin butyl ether, 2-hydroxy-2-benzoylpropane, 2-hydroxy-2-(4'-isopropyl)benzoylpropane, 4-butylbenzoyltrichloromethane, 4-phenoxybenzoyldichloromethane, methyl benzoylformate, 1,7-bis(9'-acridinyl) heptane, 9-n-butyl-3,6-bis(2'-morpholinoisobutyroyl)carbazole, 2-methyl-4,6-bis(trichloromethyl)-s-triazine, 2-phenyl-4,6-bis(trichloromethyl)-s-triazine, 2-naphthyl-4,6-bis(trichloromethyl)-s-triazine, 2,2-bis(2-chlorophenyl)-4,5,4',5'-tetraphenyl-1,2'-biimidazole, 4,4-azobisisobutyronitrile, triphenylphosphine, camphorquinone; N-1414, N-1717, N-1919, NCI-831, and NCI-930 (from ADEKA Corp.); Irgacure 379, Irgacure 819, Irgacure 784, Irgacure 250, Lucirin TPO, Irgacure OXE 01, and Irgacure OXE 02 (from BASF); TR-PBG-304, TR-PBG-305, and TR-PBG-314 (from Tronly); benzoyl peroxide; and compounds represented by the following General Formula (III). The amount of the other photopolymerization initiator(s), if used, is preferably equal to or less than 1 time, by mass, the amount of use of the compound represented by the aforementioned General Formula (I). One type of the aforementioned photopolymerization initiator may be used, or two or more types may be used in combination.

[Chem. 15]

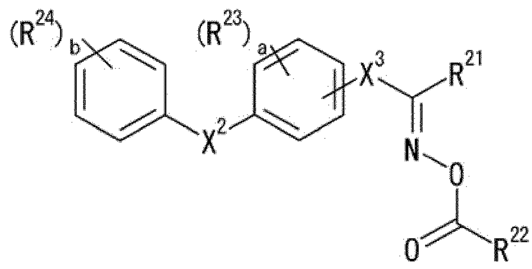

(III)

(wherein, $R^{21}$ and $R^{22}$ each independently represent a hydrogen atom, a cyano group, a $C_{1-20}$ alkyl group, a $C_{6-30}$ aryl group, a $C_{7-30}$ arylalkyl group, or a $C_{2-20}$ heterocyclic group;
$R^{23}$ and $R^{24}$ each independently represent a halogen atom, a nitro group, a cyano group, a hydroxyl group, a carboxyl group, $R^{25}$, $OR^{26}$, $SR^{27}$, $NR^{28}R^{29}$, $COR^{30}$, $SOR^{31}$, $SO_2R^{32}$, or $CONR^{33}R^{34}$, and $R^{23}$ and $R^{24}$ may be connected together to form a ring;
$R^{25}$, $R^{26}$, $R^{27}$, $R^{28}$, $R^{29}$, $R^{30}$, $R^{31}$, $R^{32}$, $R^{35}$ and $R^{34}$ each independently represent a $C_{1-20}$ alkyl group, a $C_{6-30}$ aryl group, a $C_{7-30}$ arylalkyl group, or a $C_{2-20}$ heterocyclic group;
$X^2$ represents an oxygen atom, a sulfur atom, a selenium atom, $CR^{35}R^{36}$, CO, $NR^{37}$, or $PR^{38}$;
$X^3$ represents a single bond or CO;
$R^{21}$, $R^{22}$, $R^{25}$, $R^{26}$, $R^{27}$, $R^{28}$, $R^{29}$, $R^{30}$, $R^{35}$, $R^{36}$, $R^{37}$, and $R^{38}$ each represent a $C_{1-20}$ alkyl group, a $C_{6-30}$ aryl group, or a $C_{7-30}$ arylalkyl group, and a methylene group in the alkyl group or the arylalkyl group may optionally be substituted by a halogen atom, a nitro group, a cyano group, a hydroxyl group, a carboxyl group, or a heterocyclic group, or may optionally be interrupted by -O-;
$R^{35}$, $R^{36}$, $R^{37}$, and $R^{38}$ may each independently form a ring together with one of the adjacent benzene rings;
a represents an integer from 0 to 4; and
b represents an integer from 0 to 5.)

The photosensitive composition of the present invention may further contain, as necessary, commonly-used additives, including, for example: photo/thermal acid generators; thermal polymerization inhibitors, such as p-anisole, hydroquinone, pyrocatechol, t-butylcatechol, and phenothiazine; plasticizers; adhesion accelerators; fillers; anti-foaming agents; leveling agents; surface modifiers; antioxidants; ultraviolet absorbers; dispersing aids; anti-coagulants; catalysts; curing accelerators; cross-linking agents; and thickeners.

The amount of optional components (excluding the above-mentioned other photopolymerization initiators, the alkali-developable compound optionally having an ethylenically unsaturated group, the inorganic compounds (fillers), the colorants, and the solvents) other than the present polymerizable compound having an ethylenically unsaturated bond and the compound represented by the aforementioned General Formula (I) used in the photosensitive composition of the invention is not particularly limited and can be determined as appropriate depending on the usage thereof, but the total usage amount of such optional components is preferably equal to or less than 50 parts by mass with respect to 100 parts by mass of the polymerizable compound having an ethylenically unsaturated bond.

The photosensitive composition of the invention may further contain other organic polymer(s) in addition to the polymerizable compound having an ethylenically unsaturated bond, to improve the characteristics of the cured products obtained. Examples of organic polymers include polystyrene, polymethyl methacrylate, methyl methacrylate-ethyl acrylate copolymers, poly(meth)acrylic acid, styrene-(meth)acrylic acid copolymers, (meth)acrylic acid-methyl methacrylate copolymers, ethylene-vinyl chloride copolymers, ethylene-vinyl copolymers, polyvinyl chloride resins, ABS resins, nylon 6, nylon 66, nylon 12, urethane resins, polycarbonate, polyvinyl butyral, cellulose esters, polyacrylamide, saturated polyesters, phenol resins, phenoxy resins, polyamide-imide resins, polyamic acid resins, and epoxy resins. Preferred among the above are polystyrene, (meth)acrylic acid-methyl methacrylate copolymers, and epoxy resins.

The amount of use of the other organic polymer(s), if used, is preferably from 10 to 500 parts by mass with respect to 100 parts by mass of the polymerizable compound having an ethylenically unsaturated bond.

The photosensitive composition of the invention may further employ, for example, a chain transfer agent, a sensitizer, a surfactant, a silane coupling agent, and/or a melamine compound.

Sulfur-containing compounds are generally used as the above-described chain transfer agent or sensitizer. Examples include: mercapto compounds, such as thioglycolic acid, thiomalic acid, thiosalicylic acid, 2-mercaptopropionic acid, 3-mercaptopropionic acid, 3-mercaptobutyric acid, N-(2-mercaptopropionyl)glycine, 2-mercaptonicotinic acid, 3-[N-(2-mercaptoethyl)carbamoyl]propionic acid, 3-[N-(2-mercaptoethyl)amino]propionic acid, N-(3-mercaptopropionyl)alanine, 2-mercaptoethanesulfonic acid, 3-mercaptopropanesulfonic acid, 4-mercaptobutanesulfonic acid, dodecyl (4-methylthio)phenyl ether, 2-mercaptoethanol, 3-mercapto-1,2-propanediol, 1-mercapto-2-propanol, 3-mercapto-2-butanol, mercaptophenol, 2-mercaptoethylamine, 2-mercaptoimidazole, 2-mercaptobenzimidazole, 2-mercapto-3-pyridinol, 2-mercaptobenzothiazole, mercaptoacetic acid, trimethylolpropane tris(3-mercaptopropionate), and pentaerythritol tetrakis(3-mercaptopropionate); disulfide compounds obtained by oxidizing the above mercapto compounds; iodized alkyl compounds, such as iodoacetic acid, iodopropionic acid, 2-iodoethanol, 2-iodoethanesulfonic acid, and 3-iodopropanesulfonic acid; aliphatic polyfunctional thiol compounds, such as trimethylolpropane tris(3-mercaptoisobutyrate), butanediol bis(3-mercaptoisobutyrate), hexanedithiol, decanedithiol, 1,4-dimethylmercaptobenzene, butanediol bisthiopropionate, butanediol bisthioglycolate, ethylene glycol bisthioglycolate, trimethylolpropane tristhioglycolate, butanediol bisthiopropionate, trimethylolpropane tristhiopropionate, trimethylolpropane tristhioglycolate, pentaerythritol tetrakisthiopropionate, pentaerythritol tetrakisthioglycolate, trishydroxyethyl tristhiopropionate, diethylthioxanthone, diisopropylthioxanthone, the following Compound No. C1, and trimercaptopropionate tris(2-hydroxyethyl)isocyanurate; and Karenz MT BD1, PE1, and NR1 available from Showa Denko K.K.

[Chem. 16]

Compound No. C1

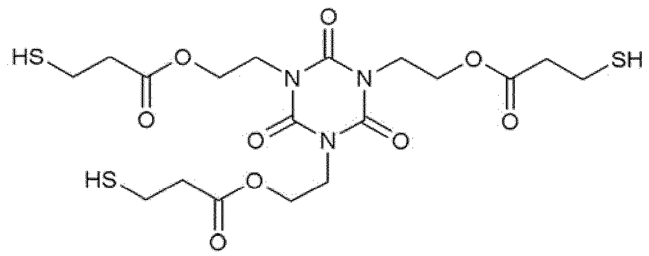

Examples of usable surfactants include: fluorine-containing surfactants, such as perfluoroalkylphosphoric esters and perfluoroalkylcarboxylic acid salts; anionic surfactants, such as higher fatty acid alkali salts, alkylsulfonic acid salts, and alkylsulfuric acid salts; cationic surfactants, such as higher amine halogen acid salts and quaternary ammonium salts; nonionic surfactants, such as polyethylene glycol alkyl ethers, polyethylene glycol fatty acid esters, sorbitan fatty acid esters, and fatty acid monoglycerides; amphoteric surfactants, and silicone surfactants. These surfactants may be used in combination.

Examples of usable silane coupling agents include those available from Shin-Etsu Chemical Co., Ltd., and among them, silane coupling agents having an isocyanate, methacryloyl, or epoxy group, such as KBE-9007, KBM-502, and KBE-403, can suitably be used.

Examples of melamine compounds include compounds wherein all or some (at least two) of the active methylol groups ($CH_2OH$ groups) in a nitrogen-containing compound, such as (poly)methylol melamine, (poly)methylol glycoluril, (poly)methylol benzoguanamine, or (poly)methylol urea, have been alkyletherified.

Examples of alkyl groups constituting the alkyl ether include methyl, ethyl, butyl, etc., which may all be the same or may be different from one another. Non-alkyletherified methylol groups may be self-condensed within a single molecule, or those of two molecules may undergo condensation to form an oligomer component.

Specifically, it is possible to use, for example, hexamethoxymethylmelamine, hexabutoxymethylmelamine, tetramethoxymethylglycoluril, or tetrabutoxymethylglycoluril.

Preferred among the above are alkyletherified melamines, such as hexamethoxymethylmelamine and hexabutoxymethylmelamine.

The photosensitive composition of the invention can be applied to supporting substrates, such as soda glass, quartz glass, semiconductor substrates, metals, paper, or plastics, using any known means for application, such as a spin coater, a roll coater, a bar coater, a die coater, a curtain coater, various printing techniques, and dipping. The photosensitive composition may temporarily be applied to a supporting substrate, such as a film, and then transferred onto another substrate. The application method therefor is not limited.

Examples of energy rays usable for curing the photosensitive composition of the invention include high-energy rays such as electron beams, X rays, ionizing radiations, and electromagnetic-wave energy with wavelengths ranging from 2000 to 7000 angstroms obtained from light sources such as ultra-high-pressure-, high-pressure-, medium-pressure-, or low-pressure mercury lamps, mercury-vapor arc lamps, xenon arc lamps, carbon arc lamps, metal halide lamps, fluorescent lamps, tungsten lamps, excimer lamps, bactericidal lamps, light-emitting diodes, CRTs, etc. Preferable light sources include ultra-high-pressure mercury lamps, mercury-vapor arc lamps, carbon arc lamps, xenon arc lamps, etc.,

that emit light in the wavelength range from 300 to 450 nm.

Using laser beams as the light source for exposure is also advantageous, because direct laser imaging, which directly forms images from digital information stored in e.g. computers without using a mask, can improve productivity as well as resolution and positional accuracy. Laser beams within the wavelength range from 340 to 430 nm can suitably be used, but various lasers that emit light within the visible to infrared region, such as excimer lasers, nitrogen lasers, argon ion lasers, helium-cadmium lasers, helium-neon lasers, krypton ion lasers, various semiconductor lasers, and YAG lasers, may also be used. In case of using these types of lasers, sensitizing dyes are added to absorb light in the visible to infrared region.

The photosensitive composition of the invention has unlimited application. It finds use in, for example: photocuring paints or varnishes; photocuring adhesives; printed boards; color filters for liquid crystal color display devices, such as color TV monitors, PC monitors, mobile data terminals, and digital cameras; color filters for CCD image sensors; electrode materials for plasma display panels; powder coatings; printing plates; adhesives; compositions for dental use; gel coats; photoresists for electronics; electroplating resists; etching resists; soldering resists; resists for manufacturing color filters of various displays or for forming structures in the manufacture of plasma display panels, electroluminescent displays, and LCDs; encapsulating compositions for electric/electronic components; magnetic recording materials; fine machine parts; waveguides; optical switches; plating masks; etching masks; color test systems; glass fiber cable coatings; screen printing stencils; materials for making a three-dimensional object by stereolithography; holographic recording materials; image recording materials; fine electronic circuits; decolorizing materials; decolorizing materials for image recording materials; decolorizing materials for image recording materials using microcapsules; photoresist materials for printed wiring boards; photoresist materials for direct imaging using UV and visible lasers; and photoresist materials or protective films used to form dielectric layers in the fabrication of multilayered printed circuit boards.

The photosensitive composition of the invention can also be used for forming spacers for LCD panels and for forming protrusions for vertical-alignment LCD elements. Particularly, the photosensitive composition is useful for simultaneously forming spacers and protrusions for vertical-alignment LCD elements.

The spacers for LCD panels are preferably produced through (1) a step of forming, on a substrate, a film consisting of the photosensitive composition of the present invention, (2) a step of irradiating the film with radiation via a mask having a predetermined pattern thereon, (3) a post-exposure baking step, (4) a post-exposure film developing step, and (5) a post-development film heating step.

The photosensitive (colored) composition of the invention which contains an ink repellent agent is also useful as a partition-wall-forming resin composition for use in inkjet processing. Such a composition is used for color filters, and preferably for partition walls for inkjet color filters particularly with profile angles of 50° or above. Fluorine-containing surfactants as well as compositions containing fluorine-containing surfactants can suitably be used as the ink repellent agent.

An optical element can be manufactured by a method of forming, on a transfer recipient, partition walls using the photosensitive composition of the invention to partition the transfer recipient, and supplying liquid droplets into the recesses formed on the partitioned transfer recipient through inkjet processing, to form image regions thereon. It is preferable that the liquid droplets contain a coloring agent and that the image regions are thus colored. A preferably-used optical element is manufactured according to the above-described optical-element manufacturing method and thus has, on a substrate, at least a group of pixels consisting of a multitude of the colored regions, and partition walls partitioning the colored regions in the group of pixels.

The photosensitive composition of the invention can also be used as compositions for protection films and insulation films, and may thus contain an ultraviolet absorber, alkylated and/or acrylated melamine, mono- or difunctional (meth)acrylate monomers containing alcoholic hydroxyl groups in their molecules, and/or silica sol.

A preferable example of a photosensitive composition for a protective film or an insulation film may be a resin composition including, as main components:

(A) a carboxyl-group-containing resin that is obtained by reacting a diol compound with a polyhydric carboxylic acid and that has a weight-average molecular weight of from 2,000 to 40,000 and an acid value of from 50 to 200 mgKOH/g;
(B) an unsaturated compound containing at least one photopolymerizable ethylenically unsaturated bond in each molecule;
(C) an epoxy compound; and
(D) a photopolymerization initiator represented by the aforementioned General Formula (I),

wherein, the composition preferably contains from 10 to 40 parts by weight of component (C) and from 0.1 to 20 parts by weight of component (D) with respect to 100 parts by weight as a total of component (A) and component (B).

The insulation film is used as an insulating resin layer in a laminate having the insulating resin layer disposed on a peelable supporting substrate. The laminate is preferably developable with an alkaline aqueous solution, and the

insulating resin layer preferably has a thickness of from 3 to 100 μm.

By including inorganic materials (inorganic compounds), the photosensitive composition of the invention can also be used as a photosensitive paste composition. The photosensitive paste composition can be used for forming baked patterns of plasma display panels, such as partition wall patterns, dielectric patterns, electrode patterns, and black matrix patterns.

Next, the compound of the present invention is described. The compound of the present invention is a novel compound represented by the aforementioned General Formula (I').

The groups/atoms given as examples in the aforementioned General Formula (I) are applicable, in the aforementioned General Formula (I'), to: the $C_{1-12}$ alkyl groups, as represented by $R^{1'}$, $R^{2'}$, $R^{3'}$, and $R^{4'}$, that are not substituted or that are substituted by a hydroxyl group, a carboxyl group, a halogen atom, a cyano group, or a nitro group; the $C_{1-8}$ alkyl groups, as represented by $R^{5'}$, $R^{6'}$, $R^{7'}$, and $R^{8'}$, that are not substituted or that are substituted by a halogen atom; the $C_{1-4}$ alkyl groups as represented by $R^{9'}$ and $R^{99'}$; the arylalkyl groups, as represented by $R^{1'}$, $R^{2'}$, $R^{3'}$, $R^{4'}$, and $R^{10'}$, that are not substituted or that are substituted by a $C_{1-4}$ alkyl group, a hydroxyl group, a carboxyl group, a halogen atom, a cyano group, or a nitro group; the $C_{2-12}$ alkenyl groups as represented by $R^{1'}$, $R^{2'}$, $R^{3'}$, and $R^{4'}$; the 3- to 6-membered heterocycles that may be formed by connecting together $R^{1'}$ and $R^{2'}$; the 3- to 6-membered rings that may be formed by connecting together $R^{3'}$ and $R^{4'}$; and halogen atoms that may substitute $R^{1'}$, $R^{2'}$, $R^{3'}$, $R^{4'}$, $R^{5'}$, $R^{6'}$, $R^{7'}$, $R^{8'}$, $R^{9'}$, and $R^{10'}$.

Compounds represented by the aforementioned General Formula (I') may be synthesized, for example, by reacting a Halogen Substance 1 and a Thiol Compound 1 according to the scheme described below.

[Chem. 17]

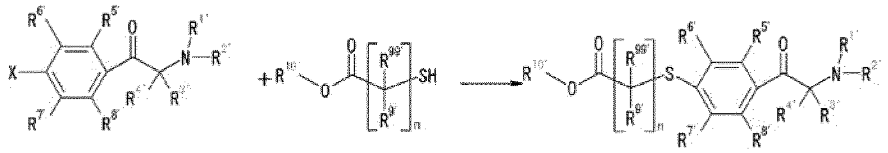

(I')

Halogen Substance 1 Thiol Compound 1

(wherein, X represents a halogen atom; and $R^{1'}$, $R^{2'}$, $R^{3'}$, $R^{4'}$, $R^{5'}$, $R^{6'}$, $R^{7'}$, $R^{8'}$, $R^{9'}$, $R^{99'}$, $R^{10'}$, and n are the same as those in the aforementioned General Formula (I').)

Examples of novel compounds represented by the aforementioned General Formula (I') include the examples given as the aforementioned Compounds Nos. 17 to 30.

The compounds represented by the aforementioned General Formula (I') may be used, for example, as radical polymerization initiators, particularly photopolymerization initiators or thermal polymerization initiators, and sensitizers, and may also be used, for example, in optical filters used for displays and optical lenses, photosensitive materials for silver-halide photography, dyes, coatings, and optical recording colorants.

## Examples

The present invention will now be described in further detail below according to Examples and Comparative Examples. The invention, however, is not to be limited to the Examples etc.

### Production Example 1: Production of Compound No. 1

1.0 eq. of Halogen Substance 1 was dissolved in 9.0 eq. of dimethyl sulfoxide, and 4.0 eq. of potassium carbonate and 1.8 eq. of Thiol Compound 1 were added, and the mixture was heated and stirred in a nitrogen atmosphere at 70°C for 10 hours. After the mixture was cooled to room temperature, the mixture was neutralized with dilute hydrochloric acid and extracted from ethyl acetate. The solvent was removed by evaporation, and the crude product was recrystallized from 1:1 methanol/ion-exchange water and was dried, to obtain Compound No. 1 of Chem. 3 (yield: 23.2%). Various analyses were conducted to verify that the obtained compound was the intended Compound No. 1. The results of analyses are shown below.

In the present Production Example, a compound in which, in Halogen Substance 1 in Chem. 17, $R^{1'}$ and $R^{2'}$ were connected together to form a morpholine ring, $R^{3'}$ and $R^{4'}$ were methyl groups, $R^{5'}$, $R^{6'}$, $R^{7'}$, and $R^{8'}$ were hydrogen, and X was bromine was used as Halogen Substance 1. Further, a compound in which, in Thiol Compound 1 in Chem. 17, $R^{9'}$, $R^{99'}$, and $R^{10'}$ were hydrogen and n was 2 was used as Thiol Compound 1.

Production Example 2: Production of Compound No. 17

1.0 eq. of Halogen Substance 1 was dissolved in 9.0 eq. of dimethyl sulfoxide, and 4.0 eq. of potassium carbonate and 1.8 eq. of Thiol Compound 1 were added, and the mixture was heated and stirred in a nitrogen atmosphere at 120°C for 20 hours. Impurities were filtered out by adding an excessive amount of toluene, and the solution was cooled to 5°C. The precipitated solid was filtered and was washed. The obtained solid was dissolved in water, the solution was neutralized with dilute hydrochloric acid and extracted from chloroform. The solvent was removed by evaporation, and the product was recrystallized from ethyl acetate and was dried, to obtain Compound No. 17 (yield: 7.6%). Various analyses were conducted to verify that the obtained compound was the intended Compound No. 17. The results of analyses are shown below.

In the present Production Example, a compound in which, in Halogen Substance 1 in Chem. 17, $R^{1'}$ and $R^{2'}$ were connected together to form a morpholine ring, $R^{3'}$ and $R^{4'}$ were methyl groups, $R^{5'}$, $R^{6'}$, $R^{7'}$, and $R^{8'}$ were hydrogen, and X was bromine was used as Halogen Substance 1. Further, a compound in which, in Thiol Compound 1 in Chem. 17, $R^{9'}$ was a methyl group, $R^{99'}$ and $R^{10'}$ were hydrogen, and n was 1 was used as Thiol Compound 1.

[Table 1]

| {H-NMR} | | |
|---|---|---|
| | Measurement solvent | Chemical shift (ppm) |
| Compound 1 | DMSO-D6 | 12.40 (s, 1H), 8.42 (d, 2H), 7.34 (d, 2H), 3.56 (t, 4H), 3.22 (t, 2H), 2.60 (t, 2H), 2.50 (s, 6H), 2.44 (t, 4H) |
| Compound 17 | CDCl3 | 8.47 (d, 2H), 7.41 (d, 2H), 3.96 (q, 1H), 3.68 (t, 4H), 2.56 (t, 4H), 1.59 (d, 3H), 1.31 (s, 6H) |

[Table 2]

| {IR} | |
|---|---|
| | Peak ($cm^{-1}$) |
| Compound 1 | 2791, 2822, 1722, 1673, 1365, 1205, 1106, 854 |
| Compound 17 | 3272, 2970, 1738, 1661, 1582, 1365, 1228, 830 |

[Table 3]

| {TG-DTA} | | |
|---|---|---|
| | Melting point (°C) | 5% Weight-loss temperature (°C) |
| Compound 1 | 112 | 235 |
| Compound 17 | 150 | 243 |

Example 1: Production of Alkali-Developable Photosensitive Resin Composition No. 1

54.84 g of SPC-1000 (acrylic resin from Showa Denko K.K.), 12.77 g of Aronix M-450 (polyfunctional acrylate from Toagosei Co., Ltd.), 0.57 g of KBE-403 (silane coupling agent from Shin-Etsu Silicone), 3.19 g of a 1% cyclohexanone solution of FZ-2122 (surfactant from Nippon Unicar Co., Ltd.), and 27.95 g of propylene glycol 1-monomethyl ether 2-acetate were mixed, and 14.805 g of the mixed solution was weighed and sampled. To this, 0.195 g of Compound No. 1 obtained in Production Example 1 was added, and the mixture was stirred thoroughly, to obtain Alkali-developable photosensitive resin composition No. 1, which is a photosensitive composition of the invention.

Example 2: Production of Alkali-Developable Photosensitive Resin Composition No. 17

Alkali-developable photosensitive resin composition No. 17, which is a photosensitive composition of the invention, was obtained in the same way as in Example 1, except that Compound No. 1 obtained in Production Example 1 was changed to Compound No. 17 obtained in Production Example 2.

Comparative Example 1: Production of Comparative Alkali-Developable Photosensitive Resin Composition No. 1

Comparative alkali-developable photosensitive resin composition No. 1 was obtained in the same way as in Example 1, except that Compound No. 1 obtained in Example 1 was changed to 2-morpholyl-2-(4'-methylmercapto)benzoylpropane.

Evaluation Examples 1 and 2 and Comparative Evaluation Example 1: Evaluation of Sublimability

The sublimability of Compound No. 1, Compound No. 17, and a comparative compound (2-morpholyl-2-(4'-methylmercapto)benzoylpropane) was evaluated as follows.

2.5 mg of the evaluated compound was kept in a nitrogen atmosphere at 150°C for 1 hour, and the percentage of weight reduction was calculated, to evaluate sublimability. The test results are shown in Table 4.

[Table 4]

| Compound | Δ Weight reduction (%) |
|---|---|
| No. 1 | 0.8 |
| No. 17 | 0.8 |
| Comparative compound | 15.2 |

From Table 4 above, it is clear that Compounds Nos. 1 and 17, which are compounds represented by the aforementioned General Formula (I), have low sublimability.

Evaluation Examples 2 and 3 and Comparative Evaluation Example 2: Evaluation of Radiation Sensitivity

The radiation sensitivity of the obtained Alkali-developable photosensitive resin compositions Nos. 1 and 17 and Comparative alkali-developable photosensitive resin composition No. 1 was evaluated as follows.

The evaluated alkali-developable photosensitive resin composition was spin-coated onto a glass substrate, and the coated glass substrate was prebaked on a hot plate at 90°C for 90 seconds, and was exposed, via a mask, by using a high-pressure mercury-vapor lamp as a light source. Development was conducted by using a 2.5 mass% sodium carbonate aqueous solution as a developer solution, and the substrate was washed thoroughly with water and was post-baked in an oven at 230°C for 30 minutes, to fix a pattern thereon. The height of the pattern was measured with a stylus profilometer (from Keyence Corporation), and the residual film rate (%), which was found as the radiation sensitivity, was calculated from the following equation. Note that A indicates that the light exposure amount was up to 200 mJ/cm$^2$, B indicates that the light exposure amount was from 200 to 500 mJ/cm$^2$, and C indicates that the light exposure amount was 500 mJ/cm$^2$, to achieve a residual film rate of 70% or higher. The test results are shown in Table 5.

Residual film rate (%) = (Height of pattern after post-baking/Initial film thickness)×100.

[Table 5]

| Alkali-developable photosensitive resin composition | Radiation sensitivity |
|---|---|
| No. 1 (Example 1) | A |
| No. 17 (Example 2) | A |
| Comparative No. 1 (Comparative Example 1) | A |

From Table 5 above, it is clear that compounds represented by the aforementioned General Formula (I) have excellent radiation sensitivity comparable to the comparative compound.

Accordingly, compounds represented by the aforementioned General Formula (I) have low sublimability while maintaining sensitivity, and are thus useful as photopolymerization initiators.

Industrial Applicability

The compound according to the invention has excellent stability and low sublimability, and generates radicals efficiently by emission lines at, for example, 365 nm (i-line), and is thus useful as a photopolymerization initiator used in a photosensitive composition.

## Claims

1. A photosensitive composition comprising
a photopolymerization initiator including a compound represented by General Formula (I) below (A), and
a polymerizable compound having an ethylenically unsaturated bond (B):

[Chem. 1]

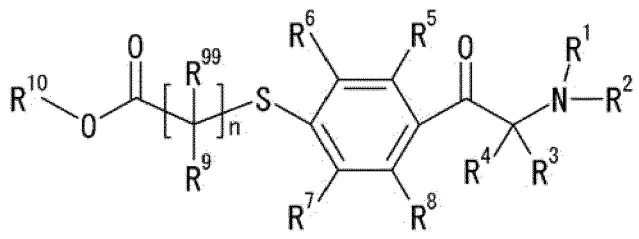

(I)

(wherein, $R^1$ and $R^2$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^1$ and $R^2$ may be connected together to form a 3- to 6-membered heterocycle;
$R^3$ and $R^4$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^3$ and $R^4$ may be connected together to form a 3- to 6-membered ring;
$R^5$, $R^6$, $R^7$, and $R^8$ each independently represent a hydrogen atom, a halogen atom, a cyano group, a nitro group, a hydroxyl group, or a $C_{1-8}$ alkyl group that is not substituted or that is substituted by a halogen atom;
$R^9$ and $R^{99}$ each independently represent a hydrogen atom, a hydroxyl group, a carboxyl group, a halogen atom, or a $C_{1-4}$ alkyl group, and the $R^9$s, and the $R^{99}$s, are the same or different from one another when n is 2 or greater;
$R^{10}$ represents a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; or a $C_{7-30}$ arylalkyl group that is not substituted or that is substituted by a $C_{1-4}$ alkyl group, a hydroxyl group, a carboxyl group, a halogen atom, a cyano group, or a nitro group;
a methylene chain in the alkyl group and the arylalkyl group may be replaced by -O- or -S-; and
n represents a number from 1 to 12.)

2. A cured product obtained by irradiating the photosensitive composition according to claim 1 with energy rays.

3. A compound represented by General Formula (I') below:

[Chem. 2]

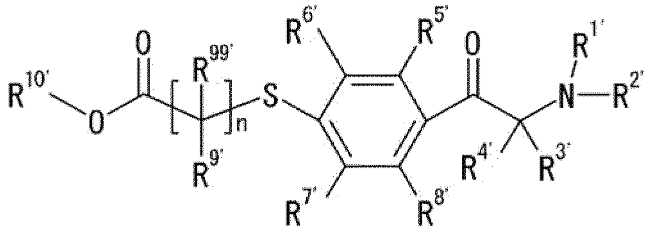

(I')

(wherein, $R^{1'}$ and $R^{2'}$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^{1'}$ and $R^{2'}$ may be connected together to form a 3- to 6-membered heterocycle;

$R^{3'}$ and $R^{4'}$ each independently represent a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; a $C_{7-30}$ arylalkyl group that is not substituted or that is substituted by a $C_{1-4}$ alkyl group, a hydroxyl group, a carboxyl group, a halogen atom, a cyano group, or a nitro group; or a $C_{2-12}$ alkenyl group, and $R^3$ and $R^{4'}$ may be connected together to form a 3- to 6-membered ring;

$R^{5'}$, $R^{6'}$, $R^{7'}$, and $R^{8'}$ each independently represent a hydrogen atom, a halogen atom, a cyano group, a nitro group, a hydroxyl group, or a $C_{1-8}$ alkyl group that is not substituted or that is substituted by a halogen atom;

$R^{9'}$ represents a hydroxyl group, a carboxyl group, a halogen atom, or a $C_{1-4}$ alkyl group, and the $R^9$'s are the same or different from one another when n is 2 or greater;

$R^{99'}$ represents a hydrogen atom, a hydroxyl group, a carboxyl group, a halogen atom, or a $C_{1-4}$ alkyl group, and the $R^{99'}$ s are the same or different from one another when n is 2 or greater;

$R^{10'}$ represents a hydrogen atom; a $C_{1-12}$ alkyl group that is not substituted or that is substituted by a hydroxyl group, a carboxyl group, a halogen atom, a cyano group, or a nitro group; a phenyl group that is not substituted or that is substituted by a $C_{1-4}$ alkyl group, a hydroxyl group, a carboxyl group, a halogen atom, a cyano group, or a nitro group; or a $C_{7-30}$ arylalkyl group that is not substituted or that is substituted by a $C_{1-4}$ alkyl group, a hydroxyl group, a carboxyl group, a halogen atom, a cyano group, or a nitro group;

a methylene chain in the alkyl group and the arylalkyl group may be replaced by -O- or -S-; and

n represents a number from 1 to 12.)

## INTERNATIONAL SEARCH REPORT

International application No.
PCT/JP2015/069399

A. CLASSIFICATION OF SUBJECT MATTER
*C08F2/48*(2006.01)i, *C07D295/10*(2006.01)i

According to International Patent Classification (IPC) or to both national classification and IPC

B. FIELDS SEARCHED

Minimum documentation searched (classification system followed by classification symbols)
C08F2/48, C07D295/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 58-157805 A (Ciba-Geigy AG.), 20 September 1983 (20.09.1983), claims; pages 21 to 24, table 1 (particularly, compounds 21, 24); pages 28 to 29, table 2 & US 4582862 A & EP 88050 A2 & CA 1198426 A | 1-3 |
| X | JP 10-069079 A (Fuji Photo Film Co., Ltd.), 10 March 1998 (10.03.1998), claims; paragraphs [0009], [0037] to [0050] (particularly, paragraph [0048], compound 77; paragraph [0049], compound 80); paragraph [0091] (table 1) (Family: none) | 1-3 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 September 2015 (14.09.15) | 29 September 2015 (29.09.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/069399

C (Continuation). DOCUMENTS CONSIDERED TO BE RELEVANT

| Category* | Citation of document, with indication, where appropriate, of the relevant passages | Relevant to claim No. |
|---|---|---|
| X | JP 2012-007070 A (Fujifilm Corp.), 12 January 2012 (12.01.2012), claims; paragraphs [0019] to [0020], [0035] (compound I-5); paragraph [0167] (compound I-2-2); paragraph [0173] (compounds (I-8-2); paragraphs [0198] to [0199] (tables 1, 2) (Family: none) | 1-3 |
| X<br>A | WO 2012/000846 A1 (BASF SE), 05 January 2012 (05.01.2012), claims; page 28, line 9 to page 30, line 21; pages 50 to 51 (table 1, compound 8); pages 52 to 53 (table 4, compound 8)<br>& JP 2013-538243 A & US 2013/0117941 A1<br>& EP 2585431 A1 & AU 2011273735 A<br>& CA 2801513 A & CN 102958909 A<br>& KR 10-2013-0027557 A & MX 2012015285 A<br>& RU 2013103401 A | 3<br>1,2 |
| X<br>A | JP 52-065240 A (Imperial Chemical Industries Ltd.), 30 May 1977 (30.05.1977), page 25 (example 30)<br>& US 4105790 A & GB 1559977 A<br>& DE 2653635 A & FR 2332747 A<br>& BE 848707 A & CH 624383 A<br>& SU 722481 A & AR 215871 A<br>& NL 7613081 A & NO 764012 A<br>& NZ 182581 A & AU 1962576 A<br>& PT 65892 A & SE 7613079 A<br>& ES 453632 A & ES 465152 A<br>& FI 763399 A & CA 1068695 A<br>& DK 531276 A & IL 50904 A<br>& IN 145003 A & ZA 7606696 A<br>& LU 76251 A & DD 127474 A<br>& PL 193861 A & AT 870976 A | 3<br>1,2 |
| A | JP 2002-069110 A (Fuji Photo Film Co., Ltd.), 08 March 2002 (08.03.2002), claims; page 35 (compound A-5); page 49 (compound C-5); page 63 (compound E-5); page 77 (compound G-5); page 91 (compound (J-5)) (Family: none) | 1-3 |
| A | JP 2007-086565 A (JSR Corp.), 05 April 2007 (05.04.2007), claims; paragraph [0007]<br>& CN 1940724 A & KR 10-2007-0034927 A | 1-3 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4321118 A
- JP 2007086565 A
- JP 2004264414 A